# EUROPEAN PATENT APPLICATION

(11) **EP 3 156 015 A1**
(43) Date of publication of application: **19.04.2017**
(21) Application number: 15806170.5
(22) Date of filing: 08.06.2015
(51) Int. Cl.: A61F 13/02, A61L 15/18, A61L 15/46, A61L 15/22

(54) **CHARCOAL DRESSING MATERIAL AND METHOD FOR PRODUCING SAME**

(30) Priority: 10.06.2014 KR 20140069957
(71) Applicant: CG Bio Co., Ltd., Jungwon-gu Seongnam-si, Gyeonggi-do 462-807 (KR)
(72) Inventor: RYU, Hyun Seung, Yongin-si Gyeonggi-do 446-727 (KR); HONG, Joon Pio, Seoul 135-836 (KR); SEO, Jun Hyuk, Seongnam-si Gyeonggi-do 463-894 (KR); PARK, Hee Jun, Seoul 135-867 (KR); PARK, Cho Hee, Seongnam-si Gyeonggi-do 462-803 (KR); HONG, Soon Gee, Jeonju-si Jeollabuk-do 560-840 (KR); JEON, Kang Jin, Seongnam-si Gyeonggi-do 462-800 (KR)
(74) Representative: Lambacher, Michael
(86) International application number: PCT/KR2015/005697
(87) International publication number: WO 2015/190768

(57) **Abstract**

Provided is a charcoal dressing material and a method of manufacturing the same. The charcoal dressing material includes: a porous substrate having a plurality of pores; and a charcoal powder adhered to a surface of the porous substrate.

## Description

### Technical Field

The present invention relates to a charcoal dressing material, and more particularly, to a charcoal dressing material and a method of manufacturing the same, which is capable of obtaining excellent deodorant and antimicrobial efficiency, and capable of reducing a production cost.

### Background Art

The skin has functions of protecting the human body from external forces, blocking water losses, controlling the body temperature, and preventing invasion of bacteria.

When defects or wounds occur to the skin, side effects such as bacterial infection, functional disorders, damage, and the like, may occur, unless a treatment is performed promptly.

That is, when a wound such as an injury, a cut, a burn, or a bedsore occurs, pain is accompanied until the wound is healed. If a treatment of healing the wound is not performed, a secondary side effect is caused, and even the life may be threatened in the worst case.

In recent years, a variety of dressing materials are available to minimize these side effects.

Korean Patent Application Publication No. 2010-0120713 discloses a dressing for distributing a decompression source at a tissue site, and for collecting and storing fluid from the tissue site. The dressing includes: an interface layer adapted to be positioned at a tissue site; an absorbent layer in fluid communication with the interface layer to absorb liquid from at least one of the interface layer and the tissue site; a diverter layer positioned between the absorbent layer and the decompression source; and a cover positioned over the diverter layer to maintain a reduced pressure at the tissue site, wherein a charcoal filter is laminated on the integrated decompression dressing, to thus form a multi-layer structure.

The charcoal filter disclosed in Korea Patent Application Publication No. 2010-0120713 has advantages of reducing an odor generated from a wound area, and an odor spreading from the integrated decompression dressing. However, since the dressing has a multi-layer structure, there is a disadvantage of causing many manufacturing expenses and since the charcoal filter is only used as a purpose for reducing an odor, there is a drawback that the dressing is irrelevant with the antimicrobial efficiency of the wound.

Thus, the present inventors have continued to conduct a research on a technology capable of obtaining excellent deodorant and antimicrobial efficiency and implemented a thin type dressing material made of a charcoal powder and a porous substrate, to thereby derive and invent a structural and methodical feature capable of reducing a production cost and allowing a charcoal powder to be in direct contact with the wound, and have completed the present invention that is more economical, applicable and competitive.

### Disclosure

### Technical Problem

To solve the above problems or defects, it is an object of the present invention to provide a charcoal dressing material and a method of manufacturing the same, which is capable of improving deodorant and antimicrobial efficiency.

It is another object of the present invention to provide a charcoal dressing material and a method of manufacturing the same, in which a thin type dressing material made of a charcoal powder and a porous substrate, is implemented, which can reduce a production cost.

### Technical Solution

In order to achieve the above object, according to an embodiment of the present invention, there is provided a charcoal dressing material comprising: a porous substrate having a plurality of pores; and a charcoal powder which is fixed to a surface of the porous substrate.

In the present invention, a portion of the charcoal powder penetrate into an inside surface of the porous substrate, and a remaining area of the charcoal powder protrudes from a surface of the porous substrate.

In the present invention, the porous substrate is implemented in a thermoplastic material.

In the present invention, the porous substrate is a woven or non-woven fabric in which fibers made of a polymer material are accumulated to form pores between the fibers.

Further, according to another embodiment of the present invention, there is provided a method of manufacturing a charcoal dressing material, the method comprising: a first step of coating a charcoal powder on a surface of a porous substrate; and a second step of applying heat only or both pressure and heat to the porous substrate to melt a surface of the porous substrate, to thereby allow a portion of the charcoal powder to penetrate into an inner surface of the melted porous substrate, and cooling the porous substrate to thus allow the charcoal powder to be fixed on a surface of the porous substrate.

In the present invention, the second step is implemented by performing a heat treatment process or a lamination process of the porous substrate on which the charcoal powder has been applied.

In the present invention, after the second step, a portion of the charcoal powder penetrates into an inside surface of the porous substrate, and a remaining area of the charcoal powder protrudes from a surface of the porous substrate.

### Advantageous Effects

As described above, the present invention has advantages to absorb an odor generated by a wound rapidly, and provide an environment to inhibit a growth of pathogenic bacteria.

According to the present invention, a dressing material on a surface of which a charcoal powder is projected by applied heat only or both and pressure, is simply manufactured to provide an advantage to improve deodorant and antimicrobial efficiency.

In the present invention, a thin type dressing material made of a charcoal powder and a porous substrate is implemented, to thus provide an effect of reducing a production cost.

### Brief Description of Drawings

FIG. 1 is a schematic cross-sectional view of a charcoal dressing material according to the present invention.
FIG. 2 is a partial schematic cross-sectional view illustrating a state a charcoal dressing material is in contact with a wound according to the present invention.
FIGS. 3A and 3B are schematic cross-sectional views for explaining a method of manufacturing a charcoal dressing material according to the present invention.
FIG. 4 is a view for explaining a process of heat-treating a porous substrate coated with a charcoal powder according to the present invention.
FIG. 5 is a view for explaining a process of laminating a porous substrate coated with a charcoal powder according to the present invention.
FIG. 6 is a partial schematic cross-sectional view illustrating a state after heat-treating or laminating a porous substrate coated with a charcoal powder according to the present invention.
FIG. 7 is a schematic plan view illustrating a state after heat-treating or laminating a porous substrate coated with a charcoal powder according to the present invention.
FIG. 8 is a schematic view for explaining a charcoal dressing material covers a wound according to the present invention.
FIG. 9 is a diagram illustrating a state of an example of mounting a cover on a charcoal dressing material according to the present invention.

### Best Mode

It should not be interpreted and understood that the terms used in the specification and in the claims are typically limited to the dictionary meanings, but the terms should be interpreted as the meanings and concepts corresponding to technical aspects of the present invention on the basis of the principle that the inventor can properly define the concept of terms to describe his or her own invention in the best way.

Accordingly, the embodiments and the configurations described herein and shown in the drawings are merely nothing but preferable embodiments of the present invention, not intended to limit the scope of the present invention, and it should be understood that a variety of equivalents and modifications may be made thereto at the point in time of filing the application.

The invention will now be described in detail referring to the drawings.

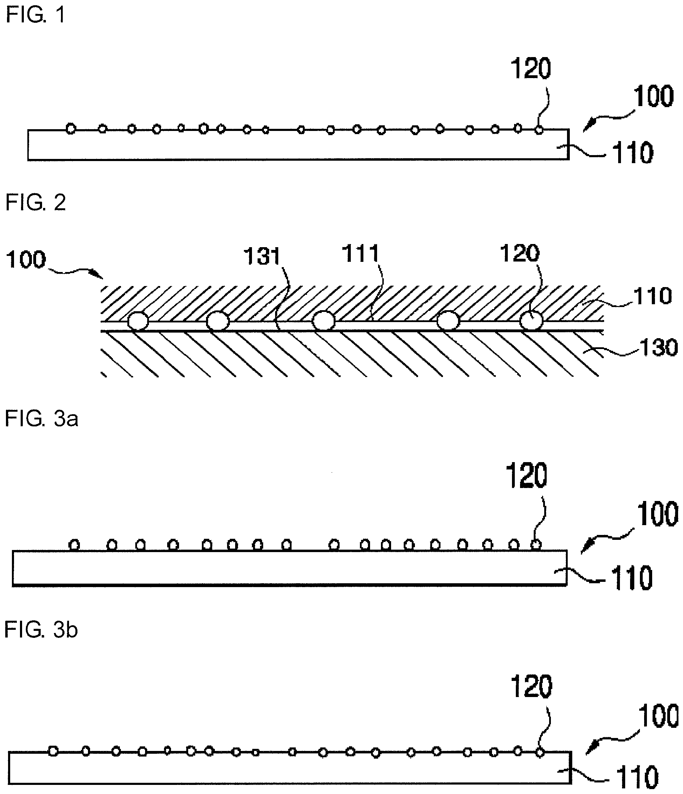

FIG. 1 is a schematic cross-sectional view of a charcoal dressing material according to the present invention. FIG. 2 is a partial schematic cross-sectional view illustrating a state a charcoal dressing material is in contact with a wound according to the present invention. FIGS. 3A and 3B are schematic cross-sectional views for explaining a method of manufacturing a charcoal dressing material according to the present invention.

Referring to FIG. 1, a charcoal dressing material 100 according to the present invention includes: a porous substrate 110 having a plurality of pores; and a charcoal powder 120 which is fixed to a surface of the porous substrate 110.

The charcoal dressing material 100 according to the invention covers a wound and is in direct contact with the wound thereby protecting the wound and promoting healing of the wound. At the same time, the charcoal powder 120, which is fixed to the surface of the porous substrate 110, is directly in contact with the wound, to thereby provide an advantage capable of seizing odors arising from the wound and allowing the wound to be antibacterial.

That is, the charcoal powder 120 absorbs the odors of the wound and prevents the offensive odors from leaking outside the charcoal dressing material 100. In addition, the charcoal powder 120 adsorbs pathogenic bacteria to be capable of inhibiting infection to the wound and growth of bacteria.

Here, the charcoal powder 120, as shown in FIG. 2, protrudes from a surface 111 of the porous substrate 110, and thus when the charcoal dressing material 100 covers the wound 130, the charcoal powder 120 is in direct contact with the surface 131 of the wound 130, rather than the surface 111 of the porous substrate 110.

Thus, the charcoal dressing material 100 of the present invention, quickly absorbs odors generated from the wound, and provides an environment which inhibits the growth of pathogenic bacteria, and thus it is possible to improve the deodorant and antimicrobial efficiency.

In addition, as the pores of the charcoal dressing material 100 according to the present invention impart breathability to a wound, an environment for breathing is given to the skin of the wound site to thus facilitate treatment and promote healing of the wound.

In the present invention, the charcoal powder 120 of the charcoal dressing material 100, may be penetrated and fixed to the inside from the surface of the porous substrate 110.

That is, when applying a pressure and heat to the porous substrate 110, after coating the charcoal powder 120 on the surface of the porous substrate 110, the surface of the porous substrate 110 is melted and a portion of the charcoal powder 120 penetrates into the inner surface of the porous substrate 110. Thereafter, when the porous substrate 110 is cooled, the portion of the charcoal powder 120 is fixed to the surface of the porous substrate 110 in a state where the portion of the charcoal powder 120 has penetrated into the inner surface of the porous substrate 110.

In addition, in the present invention, the porous substrate 110 of the charcoal dressing material 100 is preferably implemented in a thermoplastic material.

Thermoplasticity means the properties that applying heat to a thermoplastic material changes a physical temperament to a soft temper, pressing a shape frame on the thermoplastic material is imprinted as a shape after the shape frame, and cooling the heat applied to the thermoplastic material hardens the thermoplastic material as being the imprinted shape, but applying heat again to the thermoplastic material changes into the soft temper to thus be changed in another form at will.

The thermoplastic materials include polyolefins, polyesters, nylons, and a combination thereof, but are not limited to these. Polyolefins include polyethylenes, polypropylenes, polybutenes, polymethylpentenes, a copolymer thereof and a mixture thereof, and polyesters include PET (polyethylene terephthalate), PBT (polybutylene terephthalate), a copolymer thereof, and a mixture thereof.

Further, the porous substrate 110 may be a woven or non-woven fabric in which fibers made of a polymer material are accumulated to form pores between the fibers.

In addition, the charcoal dressing material 100 according to the present invention is implemented into a thin type dressing material made of a charcoal powder and a porous substrate, to thus provide an effect of reducing a production cost.

Referring to FIGS. 3A and 3B, in a method of manufacturing a charcoal dressing material according to the present invention, first, the surface of the porous substrate 110 is coated with the charcoal powder 120 (FIG. 3A).

Then, the surface of the porous substrate 110 is melted by application of only heat to the porous substrate 110, or by application of pressure and heat together thereto, and portions of the charcoal powder 120 are made to penetrate into the inner surface of the melted porous substrate 110. Then, the porous substrate 110 is cooled and thus the charcoal powder 120 is fixed on the surface of the porous substrate 110 (FIG. 3B).

Therefore, the charcoal dressing material manufacturing method according to the present invention may apply the charcoal powder 120 on the surface of the porous substrate 110 and apply heat, or both pressure and heat thereto, so that the charcoal powder 120 may be fixed to the surface of the porous substrate 110 so as to project on the surface of the porous substrate 110, to thereby provide an effect capable of manufacturing the charcoal dressing material having excellent deodorant and antimicrobial efficiency with a simple manufacturing method.

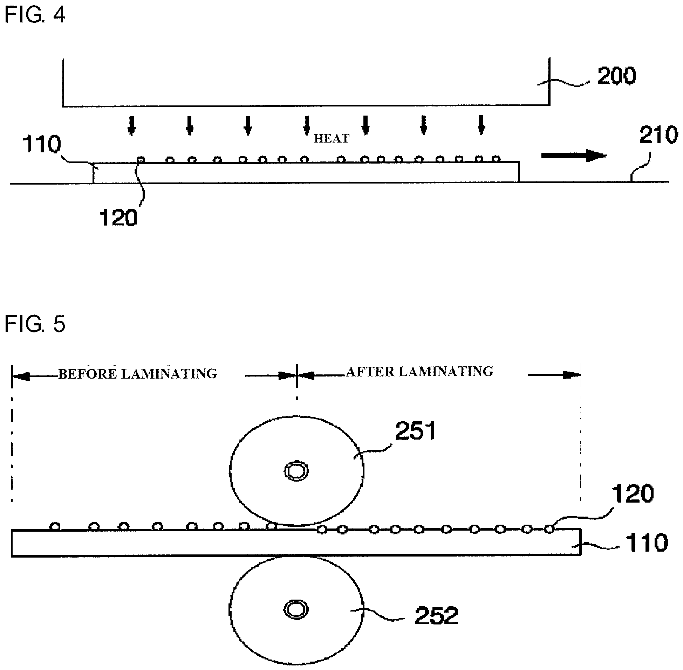

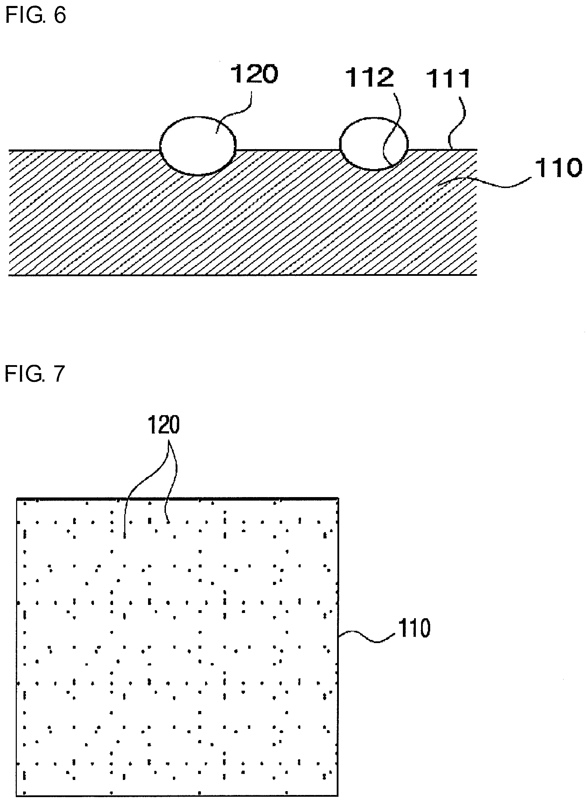

FIG. 4 is a view for explaining a process of heat-treating a porous substrate coated with a charcoal powder according to the present invention. FIG. 5 is a view for explaining a process of laminating a porous substrate coated with a charcoal powder according to the present invention. FIG. 6 is a partial schematic cross-sectional view illustrating a state after heat-treating or laminating a porous substrate coated with a charcoal powder according to the present invention. FIG. 7 is a schematic plan view illustrating a state after heat-treating or laminating a porous substrate coated with a charcoal powder according to the present invention.

In the present invention, as an example of a process of heat-treating a porous substrate coated with a charcoal powder, as shown in FIG. 4, the porous substrate 110 coated with the charcoal powder 120 is applied with heat in a furnace 200, to thus melt the surface of the porous substrate 110, so that portions of the charcoal powder 120 penetrate into the inner surface of the melted porous substrate 110.

Then, when leaving the porous substrate 110 from the furnace 200, the surface of the porous substrate 110 melted by the heat applied in the furnace 200 is cooled, and the charcoal powder 120 is fixed to the surface of the porous substrate 110.

At this point in time, the porous substrate 110 coated with the charcoal powder 120 is loaded into a side of the furnace 200 by a conveyor belt 210 and undergoes a process of melting the surface of the porous substrate 110, to then perform a heat treatment process in a configuration of a facility unloading the porous substrate 110 to the other side of the furnace 200. That is, the porous substrate 110 coated with the charcoal powder 120 is conveyed by the conveyor belt 210 in the direction of an arrow in FIG. 4. Of course, in the present invention, the heat treatment is not limited to the facility shown in FIG. 4.

Further, as an example of the process of laminating the porous substrate coated with the charcoal powder according to the present invention, as shown in FIG. 5, when a porous substrate coated with a charcoal powder is fed between rollers 251 and 252 (from the left side to the right side in FIG. 5), and the surface of the porous substrate 120 applied with only heat or both pressure and heat between the rollers 251 and 252 is melted, and some portions of the charcoal powder 110 are penetrated to the surface of the melted porous substrate 120.

Then, when the porous substrate 120 is separated from between the rollers 251 and 252, the charcoal powder 120 is fixed to the surface of the porous substrate 110, while the porous substrate 120 is cooled.

In other words, the charcoal powder is coated on the surface of the porous substrate prior to laminating, the charcoal powder is penetrated into and fixed to the inside surface of the porous substrate after laminating.

As described above, as shown in FIG. 6, after performing heat-treating or laminating the porous substrate 110 coated with the charcoal powder 120, a portion of the charcoal powder 120 penetrate into an inside surface of the porous substrate 110, and a remaining area of the charcoal powder 120 protrudes from a surface 111 of the porous substrate 110.

In other words, grooves 112 that may seat portions of the charcoal powder 120 are formed on the surface 111 of the porous substrate 110, and portions of the charcoal powder 120 are fixed into the grooves 112.

Then, since the charcoal powder 120 is coated on and fixed to the porous substrate 110, as shown in FIG. 7, the charcoal powder 120 is randomly distributed on the surface of the porous substrate 110.

Thus, when the surface of the porous substrate is melted by the heat treatment in the present invention, the charcoal particles are penetrated inside the surface of the melted porous substrate. When the porous substrate is then cooled, the charcoal particles are firmly fixed to the surface of the porous substrate. Thus, since the charcoal particles are fixed in the charcoal dressing material, although the charcoal dressing material is removed from the wound area, the charcoal particles do not remain in the wound site, to thereby prevent inflammation due to the remaining charcoal particles.

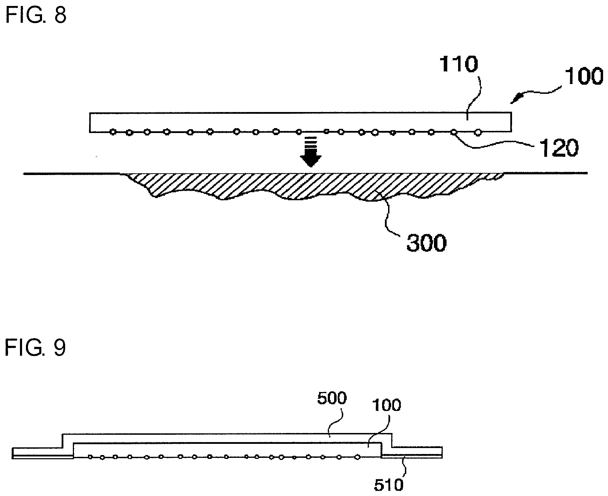

FIG. 8 is a schematic view for explaining a charcoal dressing material covers a wound according to the present invention. FIG. 9 is a diagram illustrating a state of an example of mounting a cover on a charcoal dressing material according to the present invention.

Referring to FIG. 8, the charcoal dressing material 100 according to the present invention, may cover a wound site 300, and protect and treat the wound, in which the charcoal powder 120 fixed to the surface of the porous substrate 110, is in direct contact with the wound, to thereby be capable of inhibiting infection to the wound and the growth of bacteria.

In addition, as shown in FIG. 9, an outer cover 500 provided with an adhesive 510 to facilitate adhesion to the skin may be further laminated on the charcoal dressing material 100 according to the present invention. However, the stacked state of the outer cover 500 is not limited to FIG. 9.

As described above, the present invention has been described with respect to particularly preferred embodiments. However, the present invention is not limited to the above embodiments, and it is possible for one who has an ordinary skill in the art to make various modifications and variations, without departing off the spirit of the present invention. Thus, the protective scope of the present invention is not defined within the detailed description thereof but is defined by the claims to be described later and the technical spirit of the present invention.

### Industrial Applicability

The present invention can be applied to a thin type dressing material made of a charcoal powder and a porous substrate, to thus provide an effect of reducing a production cost, and provide an advantage to improve deodorant and antimicrobial efficiency.

## Claims

1. A charcoal dressing material comprising:
a porous substrate having a plurality of pores; and
a charcoal powder which is fixed to a surface of the porous substrate.

2. The charcoal dressing material according to claim 1, wherein a portion of the charcoal powder penetrates into an inside surface of the porous substrate, and a remaining area of the charcoal powder protrudes from a surface of the porous substrate.

3. The charcoal dressing material according to claim 1, wherein the porous substrate is implemented in a thermoplastic material.

4. The charcoal dressing material according to claim 1, wherein the porous substrate is a woven or non-woven fabric in which fibers made of a polymer material are accumulated to form pores between the fibers.

5. A method of manufacturing a charcoal dressing material, the method comprising:
a first step of coating a charcoal powder on a surface of a porous substrate; and
a second step of applying heat only or both pressure and heat to the porous substrate to melt a surface of the porous substrate, to thereby allow a portion of the charcoal powder to penetrate into an inner surface of the melted porous substrate, and cooling the porous substrate to thus allow the charcoal powder to be fixed on a surface of the porous substrate.

6. The method of claim 5, wherein the second step is implemented by performing a heat treatment process or a lamination process of the porous substrate on which the charcoal powder has been applied.

7. The method of claim 5, wherein, after the second step, a portion of the charcoal powder penetrates into an inside surface of the porous substrate, and a remaining area of the charcoal powder protrudes from a surface of the porous substrate.
